(19) Europäisches Patentamt
European Patent Office
Office européen des brevets

(11) Publication number: **0 363 428 B1**

# (12) EUROPEAN PATENT SPECIFICATION

(45) Date of publication of patent specification: **09.08.95**

(51) Int. Cl.[6]: **C07C 255/39**, C07C 253/32

(21) Application number: **88906312.9**

(22) Date of filing: **02.06.88**

(86) International application number:
**PCT/US88/01873**

(87) International publication number:
**WO 88/10249 (29.12.88 88/28)**

## (54) CONVERSION OF PYRETHROID ISOMERS TO MORE ACTIVE SPECIES.

(30) Priority: **15.06.87 US 62274**

(43) Date of publication of application:
**18.04.90 Bulletin 90/16**

(45) Publication of the grant of the patent:
**09.08.95 Bulletin 95/32**

(84) Designated Contracting States:
**DE FR GB IT NL**

(56) References cited:
**EP-A- 0 022 382**
**EP-A- 0 109 113**
**GB-A- 2 064 528**

(73) Proprietor: **FMC Corporation**
**2000 Market Street**
**Philadelphia**
**Pennsylvania 19103 (US)**

(72) Inventor: **AGER, John, Winfrid**
**R.D. No. 3**
**Carson Road**
**Princeton, NJ 08540 (US)**

(74) Representative: **VOSSIUS & PARTNER**
**Postfach 86 07 67**
**D-81634 München (DE)**

**EP 0 363 428 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the transformation of pyrethroid isomers into isomers which are more pesticidally active than the starting isomers.

The pyrethroids with which the present invention is concerned are crystallizable esters having at least one asymmetric carbon atom to which an epimerizable proton is attached. The more pesticidally active pyrethroids additionally contain at least one and usually two or more other asymmetric carbon atoms and therefore comprise isomeric mixtures wherein one or more of the isomers are more pesticidally active than the others. Representative of such pyrethroids are the alpha-cyanobenzyl esters of the formula (A):

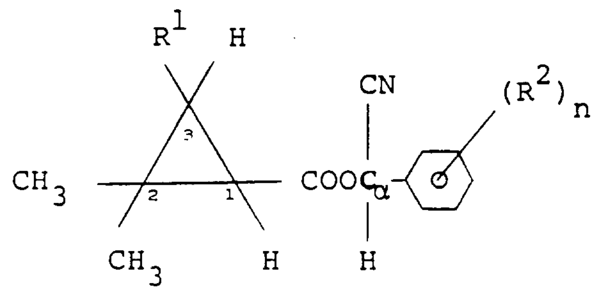

A

wherein $R^1$ is halogen, haloalkyl, alkenyl or haloalkenyl; each $R^2$ independently is halogen, alkyl, haloalkyl, alkoxy, phenyl, phenoxy, phenylalkyl, substituted phenyl and substituted phenylalkyl wherein the substituents include one or more of alkyl, halogen, haloalkyl, nitro, hydroxy and cyano; and n is 0-5, preferably 1-3. In the above formula the asymmetric carbon atoms are marked 1, 3 and alpha. All of the substituents on a host group may be the same, or the substituents may be different. Alkyl and alkoxy may contain 1-8 carbon atoms, preferably 1-4 carbon atoms. Alkenyl may comprise 2-8 carbon atoms, preferably 2-4 carbon atoms. Halogen includes fluorine, chlorine and bromine. A typical phenylalkyl group is benzyl. Substituted phenyl includes tolyl, xylyl, trichlorophenyl and trifluoromethylphenyl. Substituted phenylalkyl includes methylbenzyl, trichlorobenzyl and trifluoromethylbenzyl.

The foregoing and other pyrethroids are well known as disclosed, for example, in Kirk-Othmer, Encyclopedia of Chemical Technology, Second Edition, Vol. 13, pages 456-458, in the following U.S. Patents:

4,024,163 - Elliot et al (NRDC)
4,133,826 - Warnant et al (Roussel Uclaf)
4,136,195 - Warnant et al (Roussel Uclaf)
4,213,916 - Davies et al (Shell)
4,287,208 - Fuchs et al (Bayer)
4,308,279 - Smeltz (FMC)
4,427,598 - Mason et al (Shell)
4,512,931 - Robson (ICI)
4,544,508 - Fuchs et al (Bayer)
4,544,510 - Van Berkel et al (Shell)
4,560,515 - Stoutamire et al (Shell)
4,582,646 - Stoutamire et al (Shell)
4,670,464 - Doyle et al (ICI)
4,681,969 - Williams et al (ICI)

and in the following PCT patent publications:

WO 86/04215 - Hidasi et al (Chinoin)
WO 86/04216 - Hidasi et al (Chinoin)

Preferred pyrethroids convertible to more active isomers in accordance with the present invention are those of formula A wherein $R^1$ is dihalovinyl or tetrahalopropenyl, $R^2$ is phenoxy, and n is 1. The more preferred pyrethroids are those wherein n is 1, $R^1$ is dihalovinyl or tetrahalopropenyl and $R^2$ is phenoxy; and those wherein n is 2, $R^1$ is dihalovinyl or tetrahalopropenyl and one $R^2$ is fluorine and the other $R^2$ is phenoxy. The latter preferred compounds are isomeric mixtures having the common name "cyfluthrin" when $R^1$ is dichlorovinyl, n is 2 and one $R^2$ is fluorine. When $R^1$ is dichlorovinyl, n is 1 and $R^2$ is phenoxy, the mixtures have the common name "cypermethrin."

Cypermethrin contains four cis and four trans isomers designated I-VIII as follows:

cis isomers

I. (S)(α-cyano)(3-phenoxyphenyl)methyl 1R,cis-3-(2,2-dichloroethenyl)-2,2-dimethylcyclopropanecarboxylate (abbreviated 1R,cis S)
II. (R)(α-cyano)(3-phenoxyphenyl)methyl 1S,cis-3-(2,2-dichloroethenyl)-2,2-dimethylcyclopropanecarboxylate (abbreviated 1S,cis R)
III. (S)(α-cyano)(3-phenoxyphenyl)methyl 1S,cis-3-(2,2-dichloroethenyl)-2,2-dimethylcyclopropanecarboxylate (abbreviated 1S,cis S)
IV. (R)(α-cyano)(3-phenoxyphenyl)methyl 1R,cis-3-(2,2-dichloroethenyl)-2,2-dimethylcyclopropanecarboxylate (abbreviated 1R,cis R)

trans isomers

V. The trans isomer of I (abbreviated 1R,trans S)
VI. The trans isomer of II (abbreviated 1S,trans R)
VII. The trans isomer of III (abbreviated 1S,trans S)
VIII. The trans isomer of IV (abbreviated 1R,trans R)

Cyfluthrin and other pyrethroids to which the invention is applicable comprise similar isomeric mixtures.

It is known that the most insecticidally active isomers of the foregoing eight isomers are I and V, and that enantiomer pairs I/II (abbreviated cis-2) and V/VI (abbreviated trans-2) are more insecticidally active than the enantiomer pairs III/IV (abbreviated cis-1) and VII/VIII (abbreviated trans-1). It is extremely difficult and commercially impractical to separate the more active isomers such as I and V from the complex isomer mixtures produced in the usual pyrethroid synthesis. Accordingly, efforts to produce more pesticidally active pyrethroids have focused on techniques for converting less active isomers in the synthesis product mixtures to more active isomers, i.e., to enrich isomeric mixtures with respect to the more active isomers, thus avoiding complex resolution procedures and the loss represented by discard of less active isomers.

Nevertheless, even when the isomeric mixtures have been converted rather than resolved, the conversion procedures have not been commercially practical because of poor yields, usually due to production of undesired by-product, often comprising as many isomers as the desired product, and because of time-consuming multiple steps, high temperatures and/or the need to recover expensive reagents. In the case of cypermethrin the major by-product is (R,S)-2-oxo-1,2-bis(3-phenoxyphenyl) ethyl cis- and trans-3-(2,2-dichloroethenyl)-2,2-dimethylcyclopropanecarboxylate, an eight isomer mixture commonly called the "benzoin by-product." Similar by-products are encountered in the synthesis of other pyrethroids such as cyfluthrin. Representative of prior efforts to convert isomer mixtures to more active species are the procedures disclosed in U.S. Patents 4,213,916, 4,308,279, 4,544,510, 4,544,508, 4,512,931, 4,427,598, 4,670,646 and 4,681,969 and the two PCT patent publications cited above.

GB-A-2 064 528 discloses pesticidically more active pyrethroid isomers and a process for producing them in a high optical purity. The process essentially consists of cooling a solution which contains both the pesticidically more active and the pesticidically less active pyrethroid isomers. In this process the active isomers can be separated as crystalline solid. Numerous possible and preferred process parameters are stated, however, by choosing the most preferred embodiments the yield of pesticidically more active pyrethroid isomers at a high purity is of 96 % but the overall yield is only 56 % with 12 % conversion after carrying out the reaction for five days at 20 °C.

EP-B-0 022 382 is also directed to crystalline insecticide pyrethroid isomers and discloses a process for producing them. It is suggested to carry out a two-step process, the first step being the crystallization of the pesticidically active pyrethroid isomers from a solution containing both, the pesticidically more active and the pesticidically less active pyrethroid isomers and in a second step the remaining solution containing the pesticidically less active pyrethroid isomers is concentrated and then reacted with a base to reproduce the Starting isomeric mixture. By applying this two-step process a yield of 49 % can be achieved.

EP-A-0 109 113 also discloses a process for preparing pesticidically more active pyrethroid isomers from a mixture of pesticidically more active and pesticidically less active pyrethroid isomers. The process comprises treating a slurry of the pyrethroid isomers with an organic secondary or tertiary amine and then separating off a solid compound containing the pesticidically more active pyrethroid isomers.

It was therefore a demand in the state of the art of a process for producing pesticidically more active pyrethroid isomers at a high optical purity especially in a high yield which could be advantageously industrially used.

The present invention therefore provides a process for converting less pesticidally active isomers in a starting mixture of a crystallizable pyrethroid having an asymmetric carbon to which an epimerizable proton

is attached to more pesticidally active isomers, characterized by:

(a) forming a slurry of the starting mixture in a liquid medium consisting essentially of an aliphatic or alicyclic hydrocarbon of 5 - 16 carbon atoms in which the more pesticidally active isomers are substantially insoluble, and the ratio of starting mixture to hydrocarbon by weight is 1 : 1 to 1 : 4,

(b) contacting the slurry with a base and a catalyst, said catalyst being substantially soluble in the liquid medium and selected from a quaternary ammonium compound, a quaternary phosphonium compound and a crown ether, and the ratio of starting mixture to base by weight being 10:1 or higher and the ratio of starting mixture to catalyst by weight being from 10:1 to 100:1,

(c) agitating the resulting mixture while maintaining a temperature in the range of 5 to 35 °C, and

(d) recovering the resulting crystallized isomers.

In one aspect of the invention, single less active isomers are converted by the treatment to single more active isomers, or less active diastereomer mixtures are converted to single more active isomers, or a starting mixture of more active and less active enantiomer pairs is converted to an enantiomer pair mixture enriched, i.e., predominating, in the more active enantiomer pairs.

In other aspects of the invention the starting isomeric mixture is a mixture of all of the enantiomer pairs of cypermethrin or cyfluthrin, single pairs thereof, or any combination of the pairs such as the cis-1 and cis-2 pairs and the trans-1 and trans-2 pairs, and the product mixture contains higher proportions of the more active enantiomer pairs.

By the process of the invention pesticidally inactive or less active isomers or enantiomers are converted to active or more active isomers or enantiomers, and mixtures of both the more active and less active isomers or enantiomers are enriched in the more active isomers or enantiomers. The process is effective at room temperature range and with solvents which are usable in the preceding esterification reaction in which the pyrethroids are formed, thus presenting opportunity for avoiding solvent exchange. Moreover, the reagents for the conversion are inexpensive and by-product is substantially reduced with concomitant increased yield of more active product. The process therefore is eminently suitable for commercial production.

By-product is effectively reduced by employing as the base a weakly basic compound such as an alkali metal salt of a weak acid, and is more effectively reduced by adding an aldehyde scavenger to the reaction slurry containing the base. The aldehyde scavenger is believed to suppress the formation of benzoin ester by-product by reacting with aldehydes believed to be present as intermediates to the benzoin esters.

While the following description emphasizes application of the invention to isomers of cypermethrin and cyfluthrin, it will be understood that the invention is applicable to any crystallizable pyrethroid isomer or isomeric mixture, that is, to crystallizable pyrethroid compounds having at least one asymmetric carbon atom carrying an epimerizable proton. However, the invention is especially adapted to treatment of crystallizable pyrethroids having an epimerizable proton on an asymmetric carbon atom and a plurality of asymmetric carbon atoms. Such pyrethroids normally comprise mixtures of numerous isomers including enantiomer pairs, such as the eight isomers (four enantiomer pairs) of cypermethrin and cyfluthrin, described above. As pointed out above, the more isomers a pyrethroid comprises, the more difficult and expensive it is to produce the more active isomers or mixtures enriched therein. In this specification, "isomers" means and includes enantiomer pairs as well as individual isomers and isomer mixtures.

Accordingly, the starting material of the invention may be either a crude material, such as an unpurified reaction mixture containing crystallizable pyrethroid isomers, or the starting material may be purified so that it contains known isomers and proportions thereof. While the starting material initially may be in the liquid state it is necessary for the success of the invention that crystallization be initiated in a liquid medium so that the material is in a slurry form when contacted and agitated with the base and catalyst. Thus the starting material may either be totally solid or may be a liquid mixture in which crystallization is induced by seeding with one or more crystals of the more active isomers it is desired to produce. Preferably, the starting material is totally solid.

The liquid medium in which the slurry is formed consists essentially of an inert, nonpolar, hydrocarbon solvent in which the desired isomers are substantially insoluble. These inert hydrocarbons are aliphatic or cycloaliphatic hydrocarbons which are liquids in an ambient temperature range for plant processes, e.g., 5-35 °C, preferably 10-25 °C. The hydrocarbons contain 5-16 carbon atoms, preferably 6-8 carbon atoms, and therefore include straight chain and branched pentanes, hexanes, heptanes, octanes, the cyclic counterparts thereof, and any mixtures thereof.

Other solvents may be used with the hydrocarbons in the liquid medium provided they are not present in such amounts as will reduce or destroy the effectiveness of the treatment. For example, while some water or a polar organic liquid such as acetonitrile may be present in the liquid medium, it has been determined that polar liquids tend to inhibit the process by rendering the pyrethroids more soluble and thus

reduce the yields of the desired more active isomers. Water in major amounts is also undesirable because it decreases yield by increasing by-product. Likewise, the hydrocarbon solvent may include minor amounts of aromatic hydrocarbon components; again such components reduce the yield of useful product, principally by increasing solubility, thereby inhibiting crystallization. The liquid medium of the slurry therefore must predominately comprise an inert hydrocarbon solvent selected for substantial insolubility of the desired isomers therein.

The solvent is used in an amount which provides a fluid medium for the conversion process and such that the medium can be agitated easily. 1-4 parts by weight of solvent per part by weight of pyrethroid starting material are used, depending upon the starting material. A preferred proportion is 2-4 parts by weight of solvent per part by weight of pyrethroid.

Bases used in the process may include both strong and weak inorganic bases of which the following are representative: alkali or alkaline earth metal oxides, hydroxides, carbonates, bicarbonates, cyanides, cyanates, acetates and borates, and alkali metal fluorides such as KF. Other bases include organic amino compounds such as trialkylamine wherein the alkyl group contains 1 to 8 carbon atoms, including both straight and branched alkyl groups, such as triethylamine, and N-heterocycles such as pyridine, quinoline, pyrrole, pyrazole, pyrrolidine, and the like. Preferably the bases are basic salts of organic or inorganic acids such as sodium or potassium carbonate, bicarbonate, acetate and cyanide, and the potassium salt of 3-(2,2-dichloroethenyl)-2,2-dimethylcyclopropanecarboxylic acid. The bases may be used singly or in any mixtures of two or more thereof.

The bases preferably are added to the hydrocarbon solvent medium as solids. Aqueous solutions of one or more of the foregoing bases may be used but the amount of water contributed by the solution to the liquid medium must not be such as to impede the process or reduce the yield of crystalline product, as mentioned above. The amount of base may be varied depending on its strength and the economics of the treatment, e.g., residence time of the process. Weaker bases may require longer treatment time than stronger bases, and smaller amounts of stronger bases may permit treatment times equivalent to those required when weaker bases are used. For a base having a $pK_a$ of 9-11, 1 part by weight of base per 10 parts by weight of pyrethroid starting material will be effective and for a base having a $pK_a$ over 11, less than 1 part by weight of base per 10 parts by weight of pyrethroid will be sufficient.

To further reduce benzoin by-products it is preferred to add an aldehyde scavenger to the reaction slurry, either directly or indirectly by admixture with the base, to react with aldehydes which are believed to be present as intermediates to undesired benzoin ester by-products. Suitable aldehyde scavengers include alkali metal metabisulfites, hydrogen sulfites and hydrosulfites such as sodium metabisulfite, sodium hydrosulfite and sodium hydrogen sulfite. The aldehyde scavengers are employed in weight ratios relative to base of from 2:1 to 1:2, preferably 1:1. Preferred base/aldehyde scavenger pairs for optimizing suppression of benzoin by-products are potassium cyanide/sodium metabisulfite, sodium cyanide/sodium hydrogen sulfite, sodium cyanide/sodium hydrosulfite and potassium cyanide/potassium metabisulfite.

Useful catalysts include quaternary ammonium or phosphonium compounds and crown ethers different from the base. Suitable quaternary compounds are commercially available and include the following, either singly or in any admixture:

Methyl($C_8$-$C_{10}$-trialkyl)ammonium chloride
Benzyltributylammonium chloride
Benzyltriethylammonium chloride
Benzyltrimethylammonium chloride
Benzyltriphenylphosphonium chloride
n-Butyltriphenylphosphonium bromide
Cetyltrimethylammonium bromide
Dodecyltriphenylphosphonium bromide
Ethyltriphenylphosphonium bromide
Methyltributylammonium iodide
Methyltriphenylphosphonium bromide
Myristyltrimethylammonium bromide
Phenyltrimethylammonium bromide
Phenyltrimethylammonium tribromide
n-Propyltriphenylphosphonium bromide
Tetrabutylammonium bromide
Tetrabutylammonium chloride
Tetrabutylammonium hydrogen sulfate
Tetrabutylammonium hydroxide

Tetraethylammonium bromide
Tetramethylammonium chloride
Tetramethylammonium fluoride pentahydrate
Tetramethylammonium hexafluorophosphate
Tetramethylammonium hydroxide
Tetramethylammonium tetrafluoroborate
Tetraethylammonium chloride
Tricaprylmethylammonium chloride
Tris(3,6-dioxaheptyl)amine.

Other halides in addition to those listed may be used, such as the bromides, chlorides, and some iodides. The catalyst may also be reacted with a base to form a compound suitable for the treatment. Typical of such compounds are tricaprylmethylammonium phenolate, tricaprylmethylammonium methylate, benzyltrimethylammonium hydroxide, benzyltrimethylammonium methoxide, tetraethylammonium hydroxide, and tetrabutylammonium cyanide.

Since the shorter chain ($C_1$-$C_5$) quaternary catalysts are less soluble in the hydrocarbon solvent of the process than are the longer chain quaternaries, it is desirable to dissolve the shorter chain quaternary catalyst in an aprotic organic solvent such as an organic nitrile (acetonitrile, propionitrile, or the like) prior to addition of the catalyst to the reaction medium. The amount of aprotic solvent will be about equivalent to the weight of catalyst. Too much of the solvent may dissolve the starting pyrethroid isomers and thereby prevent formation of the reaction slurry necessary for the desired high conversion to more active isomers.

The crown ethers include 18-crown-6 and other variations thereof such as benzo-15-crown-5, 12-crown-4, 15-crown-5, dibenzo-18-crown-6, dibenzo-24-crown-8, dicyclohexano-18-crown-6, and the like. The foregoing and other crown ethers are commercially available and are reviewed in the literature such as Gokel and Durst, "Crown Ether chemistry: Principles and Applications", Aldrichimica Acta, 9(1), 3-12(1976), incorporated herein by reference.

The crown ethers and shorter chain quaternaries (including the basic adducts thereof) tend to lose their effectiveness in aqueous media. Therefore, these catalysts are preferably used when the hydrocarbon solvent liquid medium is anhydrous. The preferred catalysts are tricaprylmethylammonium chloride, tetrabutylammonium chloride or tetraethylammonium chloride in acetonitrile, tris(3,6-dioxaheptyl)amine, and 18-crown-6. The preferred liquid medium is anhydrous heptane. The preferred base is sodium cyanide.

The catalyst is used in amounts of 0.1-1.0 parts by weight of catalyst per 10 parts by weight of pyrethroid starting material, preferably 0.2-0.5 parts by weight of catalyst per 10 parts by weight of pyrethroid. The catalyst may be added to the pyrethroid slurry in a single addition or incrementally, such as about half initially and the balance over several hours, e.g., 3-8 hours later.

Sequence of addition of the reagents to the liquid medium normally is not critical; any of the reagents including the pyrethroid starting material may be present initially or added later. Moreover, base and catalyst may be added as a preformed adduct or the components may be added separately. In the case of a base-catalyst adduct, sequential addition is preferred, such as about half initially and the second half 3-8 hours later. The sequence of addition, amounts and proportions of reagents, however, may be adjusted to minimize the production of undesirable by-product such as benzoin esters. The preferred sequence of addition is to stir the pyrethroid starting material with the aldehyde scavenger in the solvent for about two hours and then add the base and catalyst to the mixture.

The slurry containing pyrethroid isomers, base and catalyst is agitated at a temperature of 5 to 35 °C for such time as to induce conversion to the desired isomers. One of the advantages of the invention is that the conversion may be carried out at conventional ambient temperature conditions of 5-35 °C, preferably 10-25 °C. Typically, the reaction mixture is agitated for 2 to 10 hours, preferably 3 to 8 hours, whereupon the crystalline products may be recovered by any conventional means, such as filtration, evaporation, decantation, centrifugation or any combination thereof. Although the reaction mixture can be cooled prior to filtration, it is preferred that the temperature be maintained throughout the process to reduce the possibility of trapping undesired impurities in the crystalline structure of the product. If desired, the product may be recrystallized one or more times to upgrade purity.

The hydrocarbon solvent is believed to be critical to the success of the process because it has been found that the more active isomers are less soluble therein than are the less active isomers, as compared with the solubilities in a base alone, such as triethylamine, or other solvent. At equilibrium, therefore, formation of the more active isomers is favored and is further promoted by removal of the solid, more active species as it is formed. Accordingly, by using a hydrocarbon as the dominant solvent in the process, the reaction is driven to produce the more active cis-2 pair at the expense of the less active cis-1 pair. Similar principles are believed applicable to other isomer mixtures, for example, the conversion of the less active

trans-1 pair to the more active trans-2 pair.

By the process of the invention, starting isomeric mixtures containing all four of the enantiomer pairs of cypermethrin or cyfluthrin, e.g., 15-25 wt. % of the cis-2 pair, and 15-25 wt. % of the trans-2 pair, the balance to make 100 wt. % being distributed between the cis-1 and trans-1 pairs, can be converted to mixtures predominating in the cis-2 and trans-2 pairs, e.g., at least about 30 wt. % of each pair, preferably to at least about 40 wt. % of each such pair. Moreover, starting mixtures of the cis-1 and cis-2 enantiomer pairs, e.g., 20-80 wt. % of cis-1 and 80-20 wt. % of cis-2, can be converted to mixtures having increased amounts of the more active cis-2 pair, e.g., 30-90 wt. % or more. Similarly, starting mixtures of the trans-1 and trans-2 enantiomer pairs, e.g., 20-80 wt. % of trans-1 and 80-20 wt. % of trans-2, are convertible to mixtures having increased amounts of the more active trans-2 pair, e.g., 30-90 wt. % or more.

The following examples further illustrate the invention. In the examples, the terms cis,trans, cis-1, cis-2, trans-1 and trans-2 refer to the isomers and enantiomer pairs of cypermethrin or cyfluthrin as defined above and as the case may be. In each case the compound name followed by the liquid chromatograph analysis (% area) is further abbreviated as follows: $C_1$ = cis-1 enantiomer pair; $C_2$ = cis-2 enantiomer pair; $T_1$ = trans-1 enantiomer pair; $T_2$ = trans-2 enantiomer pair; $B_1$ = cis-1 benzoin by-product enantiomer pair; $B_2$ = cis-2 benzoin by-product enantiomer pair; $B_3$ = trans-1 by-product benzoin enantiomer pair; and $B_4$ = trans-2 by-product benzoin enantiomer pair.

## EXAMPLE 1

Preparation with aqueous $Na_2CO_3$ of a mixture enriched in the cis-2 enantiomer pair of cypermethrin

To a stirred mixture of 10.0 grams (0.024 mole) of (R,S)-(cyano)(3-phenoxyphenyl)methyl cis-3-(2,2-dichloroethenyl)-2,2-dimethylcyclopropanecarboxylate ($C_1$ = 53.8, $C_2$ = 42.0, $T_1$ = 2.8, and $T_2$ = 1.2), in 20 g of n-heptane was added 0.1 gram (0.00025 mole) of tricaprylmethylammonium chloride (Aliquat® 336, Aldrich Chemical Co.) and 10 ml of an aqueous, 10% sodium carbonate solution. This mixture was stirred at room temperature for five hours. An additional 0.1 gram of tricaprylmethylammonium chloride was added, and the mixture was stirred at room temperature for 13 hours. The reaction mixture was filtered to yield 8.37 grams of the cis-2 enantiomer pair of (R,S)-(cyano)(3-phenoxyphenyl)methyl cis-3-(2,2-dichloroethenyl)-2,2-dimethylcyclopropanecarboxylate as a solid ($C_1$ = 4.0, $C_2$ = 94.0, $B_1$ = 1.1, $B_2$ = 0.6).

## EXAMPLE 2

Preparation with solid $Na_2CO_3$ of a mixture enriched in the cis-2 enantiomer pair of cypermethrin

A crude reaction mixture solution was assayed by liquid chromatography to contain 30.88 % (R,S)-(cyano)-(3-phenoxyphenyl)methyl cis-3-(2,2-dichloroethenyl)-2,2-dimethylcyclopropanecarboxylate in mixed heptanes. The mixed heptanes solvent was removed from a 32.4 gram sample of the solution to yield 9.96 grams of the cis isomers of cypermethrin, which was assayed by liquid chromatography to contain the following isomers: $C_1$ = 52.56, $C_2$ = 41.55, $T_1$ = 2.6, and $T_2$ = 2.7. Another 32.4 gram sample of the solution was seeded with cypermethrin crystals having a high cis isomer content (at least 50%) and stirred at room temperature for approximately 18 hours to crystallize. This mixture was treated with 0.1 gram (0.00025 mole) of tricaprylmethylammonium chloride and 0.5 gram of solid sodium carbonate. The mixture was stirred at room temperature for four hours at which time an additional 0.1 gram of tricaprylmethylammonium chloride was added. The reaction slurry was stirred for an additional 3.0 hours at room temperature. The reaction slurry was diluted with 15 ml of an aqueous solution containing 2.0 grams of concentrated hydrochloric acid to neutralize the $Na_2CO_3$ and was stirred for 10 minutes. This mixture was filtered to yield 7.06 grams of the cis-2 enantiomer pair of (R,S)-(cyano)(3-phenoxyphenyl)methyl cis-3-(2,2-dichloroethenyl)-2,2-dimethylcyclopropanecarboxylate as a solid ($C_1$ = 4.0, $C_2$ = 95.0, $T_2$ = 0.6, $B_2$ = 0.3).

## EXAMPLE 3

Preparation of a mixture enriched in the cis-2 and trans-2 enantiomer pairs of cypermethrin

A stirred mixture of 10.0 grams of n-heptane and 10.0 grams of an aqueous, 10% sodium carbonate solution was cooled to 10 °C. Seed crystals of (R,S)-(cyano)(3-phenoxyphenyl)methyl cis-3-(2,2-dichloroethenyl)-2,2-dimethylcyclopropanecarboxylate ($C_1$ = 54, $C_2$ = 42, $T_1$ = 3, $T_2$ = 1) were added. While continuing to maintain a temperature of 10 °C, a solution consisting of 10.0 grams (0.024 mole) of (R,S)-(cyano)(3-phenoxyphenyl)methyl cis,trans-3-(2,2-dichloroethenyl)-2,2-dimethylcyclopropanecarboxylate ($C_1$ = 26.7, $C_2$ = 18.9, $T_1$ = 23.0, $T_2$ = 15, and $B_2$ = 0.6), and 0.2 gram (0.005 mole) of tricaprylmethylammonium chloride in 10.0 grams of n-heptane was added dropwise during a 12 hour period to form a slurry. After complete addition, the slurry was stirred at 10 ° C for approximately 18 hours. This mixture was filtered to yield 7.5 grams of the cis-2 and trans-2 enantiomer pairs of (R,S)-(cyano)(3-phenoxyphenyl)methyl cis,trans-3-(2,2-dichloroethenyl)-2,2-dimethylcyclopropanecarboxylate as a solid ($C_1$ = 7, $C_2$ = 41, $T_1$ = 7, $T_2$ = 32, $B_1$ = 4.6, $B_2$ = 2.5 and $B_3$ = 3.8).

## EXAMPLE 4

Preparation of a mixture enriched in the trans-2 enantiomer pair of cypermethrin

A slurry of 10.0 grams (0.024 mole) of (R,S)-(cyano)(3-phenoxyphenyl)methyl trans-3-(2,2-dichloroethenyl)-2,2-dimethylcyclopropanecarboxylate ($C_1$ + $C_2$ = 1.1, $T_1$ = 55.4, and $T_2$ = 43.5), 0.1 gram (0.00025 mole) of tricaprylmethylammonium chloride, and 1.0 gram of sodium carbonate in 20 grams of n-heptane was stirred at room temperature for three hours. An additional 0.1 gram of tricaprylmethylammonium chloride was added, and the mixture was stirred for an additional three hours. The reaction mixture was diluted with 10 ml of water and was stirred for 15 minutes. The resultant mixture was filtered. The filter cake was washed with n-heptane to yield 9.17 gram of the trans-2 enantiomer pair of (R,S)-(cyano)(3-phenoxyphenyl)methyl trans-3-(2,2-dichloroethenyl)-2,2-dimethylcyclopropanecarboxylate ($T_1$ = 1.7 and $T_2$ = 97.5).

## EXAMPLE 5

Preparation with solid $K_2CO_3$ of a mixture enriched in the cis-2 enantiomer pair of cypermethrin

A slurry of 10.0 grams (0.024 mole) of solid (R,S)-(cyano)(3-phenoxyphenyl)methyl cis-3-(2,2-dichloroethenyl)-2,2-dimethylcyclopropanecarboxylate ($C_1$ = 51.7, $C_2$ = 46.8, and $T_1$ + $T_2$ = 1.5), 0.25 gram (0.00095 mole) of 18-crown-6, and 1.0 gram (0.0072 mole) of potassium carbonate in 20.0 grams of n-heptane was stirred at room temperature for approximately 18 hours. Dilute hydrochloric acid was added to the reaction mixture to neutralize the potassium carbonate. This mixture was stirred at room temperature for two days. The mixture was filtered, and the filter cake was dried to yield 8.72 grams of the cis-2 enantiomer pair of (R,S)-(cyano)(3-phenoxyphenyl)methyl cis-3-(2,2-dichloroethenyl)-2,2-dimethylcyclopropanecarboxylate ($C_1$ = 3.8, and $C_2$ = 94.9).

## EXAMPLE 6

Preparation of a mixture enriched in the cis-2 enantiomer pair of cyfluthrin

A slurry of 5.0 grams (0.012 mole) of solid (R,S)-(cyano)(4-fluoro-3-phenoxyphenyl)methyl cis-3-(2,2-dichloroethenyl)-2,2-dimethylcyclopropanecarboxylate ($C_1$ = 49.2 and $C_2$ = 50.8), 0.5 gram (0.0047 mole) of sodium carbonate, and 0.05 gram (0.00012 mole) of tricaprylmethylammonium chloride in 10.0 grams of n-heptane was stirred at room temperature for four hours. An additional 0.05 gram of tricaprylmethylammonium chloride was added, and the slurry was stirred at room temperature for approximately 13 hours. The basic reaction mixture was neutralized with dilute hydrochloric acid and was stirred for approximately 30 minutes. This mixture was filtered, and the filter cake was dried to yield 4.45 grams of the cis-2 enantiomer pair of (R,R)-(cyano)(4-fluoro-3-phenoxyphenyl)methyl cis-3-(2,2-dichloroethenyl)-2,2-dimethylcyclopropanecarboxylate ($C_1$ = 0.1 and $C_2$ = 99.8).

## EXAMPLE 7

Preparation with triethylamine of a mixture enriched in the cis-2 enantiomer pair of cypermethrin

A slurry of 10.0 grams (0.024 mole) of (R,S)-(cyano)(3-phenoxyphenyl)methyl cis-3-(2,2-dichloroethenyl)-2,2-dimethylcyclopropanecarboxylate ($C_1$ = 53.8, $C_2$ = 42.0, $T_1$ = 2.8, $T_2$ = 1.2), 0.1 gram (0.00025 mole) of tricaprylmethylammonium chloride, and 8.0 grams (0.079 mole) of triethylamine in 20 grams of n-heptane was stirred at room temperature for 5.5 hours. An additional 0.1 gram of tricaprylmethylammonium chloride was added, and the mixture was stirred for an additional 17 hours. The slurry

was filtered and the filter cake was washed with n-heptane. The filter cake was dried to yield 7.78 grams of the cis-2 enantiomer pair of (R,S)-(cyano)(3-phenoxyphenyl)methyl cis-3-(2,2-dichloroethenyl)-2,2-dimethylcyclopropanecarboxylate ($C_1$ = 3.6, $C_2$ = 91.0, $T_2$ = 0.9).

EXAMPLE 8

Preparation of a mixture enriched in the trans-2 enantiomer pair of cyfluthrin

A slurry of 5.0 grams (0.012 mole) of solid (R,S)-(cyano)(4-fluoro-3-phenoxyphenyl)methyl trans-3-(2,2-dichloroethenyl)-2,2-dimethylcyclopropanecarboxylate ($T_1$ = 71.0, $T_2$ = 28.0), 0.5 gram (0.0036 mole) of potassium carbonate, and 0.05 gram (0.00012 mole) of tricaprylmethylammonium chloride in 10.0 grams of n-heptane was stirred at room temperature for approximately 17 hours. The basic mixture was neutralized with dilute hydrochloric acid and stirred for approximately 30 minutes. This mixture was filtered, and the filter cake was dried to yield 4.82 grams of the trans-2-enantiomeric pair of (R,S)-(cyano)-(4-fluoro-3-pnenoxyphenyl)methyl trans-3-(2,2-dichloroethenyl)-2,2-dimethylcyclopropanecarboxylate ($T_1$ = 4.6, $T_2$ = 94.0).

EXAMPLE 9

Preparation of a mixture enriched in the 1R,cis S bromo analog of isomer I of cypermethrin from a mixture of diastereomers

A solution of 9.4 grams (0.019 mole) of (R,S)-(cyano)(3-phenoxyphenyl)methyl 1R,cis-3-(2,2-dibromoethenyl)-2,2-dimethylcyclopropanecarboxylate (1R,cis S = 46.0%, 1R,cis R = 52.0%) in 18 grams of n-heptane was stirred at room temperature until a slurry formed. To this slurry was added 0.2 gram (0.00076 mole) of 18-crown-6 and 1.0 gram (0.0072 mole) of potassium carbonate. This mixture was stirred at room temperature for approximately 20 hours. A sample of the solid submitted to gas chromatographic analysis (area %) was found to contain 87.8% (S)-(cyano)(3-phenoxyphenyl)methyl 1R,cis-3-(2,2-dibromoethenyl)-2,2-dimethylcyclopropanecarboxylate and 6.9% of the 1R,cis R isomer.

EXAMPLE 10

Preparation of the cis-2 enantiomer pair of (R,S)-(cyano)(3-phenoxyphenyl)methyl cis-3-(2,2-dichloroethenyl)-2,2-dimethylcyclopropanecarboxylate using potassium fluoride and tricaprylmethylammonium chloride

A slurry of 10.0 grams (0.024 mole) of (R,S)-(cyano)(3-phenoxyphenyl)methyl cis-3-(2,2-dichloroethenyl)-2,2-dimethylcyclopropanecarboxylate ($C_1$ = 53.9, $C_2$ = 44.4, $T_1$ and $T_2$ = 1.7), 0.1 gram (0.00025 mole) of tricaprylmethylammonium chloride, and 1.0 gram (0.02 mole) of potassium fluoride in 20 grams of n-heptane was stirred at room temperature for two hours. The reaction mixture was neutralized with dilute hydrochloric acid and was stirred for approximately 30 minutes. This mixture was filtered, and the filter cake was dried to yield 8.84 grams of the cis-2 enantiomer pair of (R,S)-(cyano)(3-phenoxyphenyl)methyl cis-3-(2,2-dichloroethenyl)-2,2-dimethylcyclopropanecarboxylate ($C_1$ = 7.0, $C_2$ = 90.0).

EXAMPLES 11-50

The appended tables illustrate other conditions effective for practice of the invention. Table 1 summarizes conditions and results for a process conducted substantially as described in Example 1 with the variations as indicated. Tables 2 and 3 have the same relationship to Examples 3 and 4, respectively. "Benzoin" in the tables means the benzoin by-product described above in this specification. "NR" means not reported.

TABLE 1

Preparation of Mixtures Enriched in Cis-2 Enantiomer Pair of Cypermethrin

| Exp. No. | Catalyst[a] Type | Catalyst[a] (g) | Solvent Type[b] | Base Type[c] | Reaction Time (Hr) | Starting Material Analysis[d] | AMT (g) | Product Analysis (Area % HPLC) $C_1$ | $C_2$ | Trans Total | Benzoin Total |
|---|---|---|---|---|---|---|---|---|---|---|---|
| 11 | A | 0.1 | A | A | 2.0 | A | e | 17.8 | 80.0 | 2.3 | NR |
| | | | | | 4.0 | | e | 9.8 | 87.6 | 2.6 | NR |
| | | | | | 6.0 | | e | 7.3 | 90.4 | 2.3 | NR |
| | | | | | 8.0 | | 8.9 | 6.7 | 92.3 | NR | NR |
| 12 | A | 0.1 | A | B | 18.0 | B | 8.7 | 6.2 | 92.6 | 0.5 | NR |
| 13 | A | 0.1 | B | C | 18.0 | B | 8.9 | 5.8 | 92.2 | 0.5 | NR |
| 14 | A | 0.2 | C | D | 17.5 | C | 7.7 | 3.9 | 86.0 | 1.2 | 1.2 |
| 15 | A | 0.2 | A | E | 17.0 | C | 8.5 | 4.0 | 93.5 | 2.0 | NR |
| 16 | See Base | | A | F | 5.5 | C | - | 26.0 | 71.0 | NR | NR |
| | | | | | 11.0 | | - | 22.0 | 75.0 | NR | 1.3 |
| | | | | | 23.0 | | 8.4 | 5.0 | 92.0 | NR | 2.6 |
| 17 | A | 0.2 | A | G | 18.0 | C | 9.1 | 7.1 | 89.2 | NR | 2.5 |
| 18 | A | 0.2 | A | H | 17.5 | C | 8.1 | 5.0 | 92.0 | 0.8 | NR |
| 19 | B | 0.5 | A | C | 18.0 | D | 8.4 | 6.0 | 93.7 | NR | 0.2 |
| 20 | B | 0.5 | A | A | 18.0 | D | 8.9 | 6.6 | 92.4 | NR | 0.3 |
| 21 | B | 0.5 | A | I | 18.0 | D | 8.4 | 19.0 | 80.5 | NR | NR |
| 22 | B | 0.5 | A | J | 18.0 | D | 8.9 | 5.8 | 93.6 | NR | NR |
| 23 | C | 0.5 | A | C | 18.0 | D | 8.1 | 22.0 | 77.0 | NR | NR |
| 24 | C | 0.5 | A | A | 18.0 | D | 8.5 | 18.0 | 80.0 | NR | NR |
| 25 | C | 0.5 | A | K | 20.0 | D | 8.0 | 7.0 | 89.0 | NR | 2.0 |
| 26 | D | 0.4 | A | L | 8.0 | D | 9.9 | 19.8 | 71.8 | 0.6 | NR |
| 27 | E | 0.2 | A | L | 24.0 | C | 7.8 | 5.0 | 92.6 | 1.4 | NR |
| 28 | F | 0.4 | A | L | 19.0 | C | 8.3 | 4.0 | 79.0 | NR | NR |

TABLE 1 (continued)

Preparation of Mixtures Enriched in
Cis-2 Enantiomer Pair of Cypermethrin

| Exp. No. | Catalyst[a] Type | Catalyst[a] (g) | Solvent Type[b] | Base Type[c] | Reaction Time (Hr) | Starting Material Analysis[d] | AMT (g) | Product Analysis (Area % HPLC) $C_1$ | $C_2$ | Trans Total | Benzoin Total |
|---|---|---|---|---|---|---|---|---|---|---|---|
| 29 | D | 0.2 | D | M | 22.0 | E | NR | 8.8 | 86.0 | 2.0 | 2.2 |
| 30 | D | 0.2 | D | N | 4.0 | E | NR | 11.6 | 82.0 | 2.2 | 3.1 |
| 31 | D | 0.2 | D | V | 22.0 | E | NR | 9.3 | 82.0 | 2.1 | 4.8 |
| 32 | D | 0.4 | A | O | 21.0 | F | NR | 8.6 | 88.0 | 2.1 | 0.1 |
| 33 | D | 0.1 | D | P | 7.0 | E | NR | 10.4 | 83.8 | 2.6 | 2.3 |
| 34 | D | 0.2 | D | Q | 18.0 | E | NR | 9.0 | 86.0 | 2.0 | 2.0 |
| 35 | D | 0.2 | D | R | 18.0 | E | NR | 10.0 | 84.0 | 2.0 | 2.3 |
| 36 | E | 0.28 | D | P | 18.0 | E | NR | 9.8 | 86.0 | 1.9 | 1.1 |
| 37 | D | 0.2 | D | S | 17.0 | E | NR | 17.0 | 80.0 | 2.0 | NR |
| 38 | G | 0.2 | D | P | 18.0 | E | NR | 9.0 | 88.0 | 2.0 | NR |
| 39 | G | 0.2 | D | T | 46.0 | E | NR | 8.2 | 87.0 | 2.0 | NR |
| 40 | D | 0.2 | D | T | 24.0 | E | NR | 7.7 | 89.0 | 2.1 | 0.1 |
| 41 | D | 0.2 | D | U | 26.0 | E | NR | 9.0 | 85.0 | 2.1 | 3.0 |
| 42 | D | 0.2 | D | W | 18.0 | E | NR | 10.8 | 86.0 | 2.4 | 0.5 |
| 43 | D | 0.2 | D | X | 18.0 | E | NR | 9.0 | 86.0 | 1.7 | 1.8 |

a. Catalyst

A = Tricaprylmethylammonium chloride
B = 18-crown-6
C = Tris(3,6-dioxaheptyl)amine
D = Tetrabutylammonium chloride/acetonitrile (50/50 mixture)
E = Tetrabutylammonium chloride
F = Tetrabutylammonium chloride/methanol (50/50 mixture)
G = Tetraethylammonium chloride/acetonitrile (50/50 mixture)

b. Solvent

A = n-heptane (20 grams)
B = n-octane (20 grams)
C = n-pentane (20 grams)
D = n-heptane (10 grams)

c. Base (1.0 gram except as indicated)

A = Solid potassium cyanide
B = Solid calcium hydroxide
C = Solid potassium carbonate
D = Aqueous, 10% sodium carbonate solution (10 ml)
E = Aqueous, 10% sodium carbonate/sodium bicarbonate - pH 9.5 (10 ml)
F = Catalyst/base compound: tricaprylmethylammonium phenolate (0.1 g)
G = Solid sodium cyanide
H = Borate buffer solution - pH 10 (VWR scientific) (10 ml)
I = Potassium acetate
J = Potassium cyanate
K = Potassium phenoxide
L = Solid sodium carbonate
M = Solid sodium acetate (0.5 gram)
N = Solid potassium cyanate (0.5 gram)
O = Potassium cyanide (2.2 grams)/sodium metabisulfite (1.0 gram)
P = Solid sodium cyanide (0.5 gram)
Q = Sodium cyanide (0.5 gram)/sodium sulfite (0.5 gram)
R = Sodium cyanide (0.5 gram)/sodium bicarbonate (0.5 gram)
S = Sodium cyanide (0.5 gram)/sodium hydrogen sulfite (0.5 gram)
T = Sodium cyanide (0.5 gram)/sodium metabisulfite (0.5 gram)
U = 3-(2,2-Dichloroethenyl)-2,2-dimethylcyclopropanecarboxylic acid, potassium salt (0.5 gram)
V = Solid potassium acetate (0.5 gram)
W = Sodium cyanide (0.5 gram)/sodium hydrosulfite (0.5 gram)
X = Sodium cyanide (0.5 gram)/sodium sulfate (0.5 gram)

d. Starting Material Analysis - area % HPLC (10.0 g starting material in Exp. 11-28, 5.0 g in Exp. 29-43)

A. $C_1 = 54.1$; $C_2 = 44.2$; $T_1+T_2 = 1.7$
B. $C_1 = 52.9$; $C_2 = 45.1$; $T_1+T_2 = 2.0$
C. $C_1 = 53.8$; $C_2 = 42.0$; $T_1+T_2 = 4.0$
D. $C_1 = 51.7$; $C_2 = 46.8$; $T_1+T_2 = 1.5$
E. $C_1 = 51.9$; $C_2 = 47.1$, $T_1+T_2 = 2.0$
F. Same as E but 10 g starting material used

e. Gas chromatographic analysis (area %) rather than HPLC.

TABLE 2

Preparation of Mixtures Enriched in Cis-2 and Trans-2 Enantiomer Pairs of Cypermethrin

| Exp. No. | Catalyst[a] (g) | Solvent[b] Type | Solvent[b] (g) | Base[c] | Reaction Time (Hr) | Starting Material Analysis[d] | AMT (g) | Product Analysis (Area % HPLC Normalized) $C_1$ | $C_2$ | $T_1$ | $T_2$ | Total Benzoin |
|---|---|---|---|---|---|---|---|---|---|---|---|---|
| 44 | 0.2 | A | 5 | A | 40 | A | NR | 17.4 | 27.9 | 12.0 | 27.6 | 15.1 |
| 45 | 0.2 | B | 20 | A | 17 | A | NR | 15.4 | 26.6 | 13.1 | 28.3 | 16.6 |
| 46 | 0.4 | C | 20 | B | 72 | B | 9.64 | 5.0 | 41.0 | 3.0 | 47.7 | 1.5 |
| 47 | 0.4 | C | 20 | C | 18 | B | NR | 5.8 | 41.0 | 3.8 | 47.6 | 0.5 |

a. Catalyst: Tricaprylmethylammonium chloride (Exp. No. 44, 45) Tetrabutylammonium chloride/acetonitrile, 50/50 (Exp. No. 46, 47)

b. Solvents

A = n-Pentane

B = 19.0 g of n-pentane and 1.0 g of methanol

C = n-heptane

c. Base: A = Aqueous, 10% sodium carbonate solution (10 ml) - 1.0 g

B = Potassium cyanide (1.0 g)/sodium metabisulfite (1.0 g)

C = Potassium cyanide (1.0 g)/potassium metabisulfite (1.2 g)

d. Analysis (area % by HPLC) 10.0 g of starting material used in Ex. 45, 12.6 g in Ex. 44; 10.0 g in Ex. 46 and 47

Ex. 44 and 45

A = $C_1$ = 26.7; $C_2$ = 18.9; $T_1$ = 23.0; $T_2$ = 16.0; Total Benzoins = 0.6

Ex. 46 and 47

B = $C_1$ = 26.6; $C_2$ = 22.7; $T_1$ = 27.6; $T_2$ = 20.7

TABLE 3

Preparation of Mixtures Enriched in Trans-2 Enantiomer Pair of Cypermethrin

| Exp. No. | Catalyst[a] (g) | Solvent[b] (g) | Base Type[c] | Base (g) | Reaction Time (Hr) | Starting Material Analysis[d] | AMT (g) | Product Analysis (Area % HPLC) Total cis | $T_1$ | $T_2$ | Total Benzoin |
|---|---|---|---|---|---|---|---|---|---|---|---|
| 48 | 0.4 | 40 | A | 1.0 | 18 | A | 18.3 | NR | 7.1 | 90.1 | 1.4 |
| 49 | 1.0 | 200 | B | 10 | 13 | B | 89.2 | NR | 9.0 | 91.0 | NR |
| 50 | 1.0 | 200 | B | 10 | 13 | B | 94.5 | NR | 2.8 | 96.3 | NR |

a. Catalyst: Tricaprylmethylammonium chloride

b. Solvent: n-heptane

c. Base

A = Aqueous solution of sodium carbonate and sodium bicarbonate; pH 9.0-9.5; 10 ml.

B = Solid sodium carbonate

d. Starting material analysis

A = 20.0 grams; total cis = NR; $T_1$ = 56.1; $T_2$ = 41.0

B = 100.0 grams; Total cis = 1.1; $T_1$ = 55.4; $T_2$ = 43.5

## Claims

1. A process for converting less pesticidally active isomers in a starting mixture of a crystallizable pyrethroid having an asymmetric carbon to which an epimerizable proton is attached to more pesticidally active isomers,
characterized by:
   (a) forming a slurry of the starting mixture in a liquid medium consisting essentially of an aliphatic or alicyclic hydrocarbon of 5 - 16 carbon atoms in which the more pesticidally active isomers are substantially insoluble, and the ratio of starting mixture to hydrocarbon by weight is 1 : 1 to 1 : 4,
   (b) contacting the slurry with a base and a catalyst, said catalyst being substantially soluble in the liquid medium and selected from a quaternary ammonium compound, a quaternary phosphonium compound and a crown ether, and the ratio of starting mixture to base by weight being 10:1 or higher and the ratio of starting mixture to catalyst by weight being from 10:1 to 100:1,
   (c) agitating the resulting mixture while maintaining a temperature in the range of 5 to 35 °C, and
   (d) recovering the resulting crystallized isomers.

2. The process of claim 1 characterized in that the starting mixture comprises the more active and less active enantiomers or cypermethrin and the resulting crystalline product predominately comprises the more active enantiomers.

3. The process of claim 2 characterized in that the starting mixture comprises the four enantiomer pairs of cypermethrin and the resulting crystalline product predominately comprises the cis-2 and trans-2

enantiomer pairs.

4. The process of claim 2 characterized in that the starting mixture comprises the cis-1 and cis-2 enantiomer pairs of cypermethrin and the resulting crystalline product predominately comprises the cis-2 enantiomer pair.

5. The process of claim 2 characterized in that the starting mixture comprises the trans-1 and trans-2 enantiomer pairs or cypermethrin and the resulting crystalline product predominately comprises the trans-2 enantiomer pair.

6. The process of claim 1 characterized in that the starting mixture comprises the more active and less active enantiomers of cyfluthrin and the resulting crystalline product predominately comprises the more active enantiomers.

7. The process of claim 6 characterized in that the starting mixture comprises the four enantiomer pairs of cyfluthrin and the resulting crystalline product predominately comprises the cis-2 and trans-2 enantiomer pairs.

8. The process of claim 6 characterized in that the starting mixture comprises the cis-1 and cis-2 enantiomer pairs of cyfluthrin and the resulting crystalline product predominately comprises the cis-2 enantiomer pair.

9. The process of claim 6 characterized in that the starting mixture comprises the trans-1 and trans-2 enantiomer pairs of cyfluthrin and the resulting crystalline product predominately comprises the trans-2 enantiomer pair.

10. The process of claim 1 characterized in that the starting mixture comprises the more active and less active enantiomers of (cyano)(3-phenoxyphenyl)methyl 3-(2,2-dibromoethenyl)-2,2-dimethylcyclopropanecarboxylate and the resulting crystalline product predominately comprises the more active enantiomers.

11. The process of claim 10 characterized in that the starting mixture comprises the 1R,cis S and 1R,cis R isomers of (cyano)(3-phenoxyphenyl)methyl 3-(2,2-dibromoethenyl)-2,2-dimethylcyclopropanecarboxylate and the resulting product predominately comprises the 1R,cis S isomer.

12. The process of claim 1 characterized in that the base is a basic salt of an organic or inorganic acid.

13. The process of claim 12 characterized in that the base is an aqueous solution of a basic salt of an organic or inorganic acid.

14. The process of claim 12 characterized in that the base is a solid.

15. The process of claim 1 characterized in that the slurry is substantially anhydrous.

16. The process of claim 1 characterized in that the base is a solid alkali or alkaline earth metal oxide, hydroxide, carbonate, bicarbonate, cyanide, cyanate, acetate or borate, an aqueous solution of one or more thereof, or a trialkyl amine.

17. The process of claim 1 characterized in that an adduct of the base and catalyst are formed prior to contact with the slurry in step (b).

18. The process of claim 1 characterized in that the starting mixture comprises the cis-1 and cis-2 enantiomer pairs of cypermethrin, the solvent is at least one aliphatic or cycloaliphatic hydrocarbon of 5-16 carbon atoms, the base is a basic salt of an organic or inorganic acid, and the catalyst is tricaprylmethylammonium chloride.

19. The process of claim 18 characterized in that the solvent is heptane and the basic salt is an alkali metal carbonate or cyanide.

20. The process of claim 1 characterized in that the slurry is formed by seeding a solution of the starting mixture with at least one crystal of the more pesticidally active isomers.

21. The process of claim 1 characterized in that the starting mixture comprises the four enantiomer pairs of cypermethrin, the solvent is at least one aliphatic or cycloaliphatic hydrocarbon of 5-16 carbon atoms, the base is a basic salt of an organic or inorganic acid, and the catalyst is tricaprylmethylammonium chloride.

22. The process of claim 21 characterized in that the solvent is heptane and the basic salt is an alkali metal carbonate or cyanide.

23. The process of claim 1 characterized in that the starting mixture comprises the trans-1 and trans-2 enantiomer pairs of cypermethrin, the solvent is at least one aliphatic or cycloaliphatic hydrocarbon of 5-16 carbon atoms, the base is a basic salt of an organic or inorganic acid, and the catalyst is tricaprylmethylammonium chloride.

24. The process of claim 23 characterized in that the solvent is heptane and the basic salt is an alkali metal carbonate or cyanide.

25. The process of claim 1 characterized in that the starting mixture comprises the four enantiomer pairs of cypermethrin or cyfluthrin wherein the amounts of the cis-2 and trans-2 enantiomer pairs each range from 15 to 25 wt. % and the crystalline product comprises at least 30 wt. % of each of the cis-2 and trans-2 enantiomer pairs.

26. The process of claim 1 characterized in that the starting mixture comprises 20-80 wt. % of the cis-1 and 80-20 wt. % of the cis-2 enantiomer pairs of cypermethrin or cyfluthrin and the crystalline product comprises at least 30-90 wt. % of the cis-2 pair.

27. The process of claim 1 characterized in that the starting mixture comprises a mixture of 20-80 wt. % of the trans-1 and 80-20 wt. % of the trans-2 enantiomers of cypermethrin or cyfluthrin and the crystalline product comprises at least 30-90 wt. % of the trans-2 pair.

28. The process of claim 1 characterized in that, in step (b), the slurry containing base and catalyst is contacted with an aldehyde scavenger.

29. The process of claim 28 characterized in that the aldehyde scavenger is an alkali metal metabisulfite, hydrogen sulfite or hydrosulfite.

30. The process of claim 28 characterized in that the base is an alkali metal cyanide and the catalyst is a tetraalkyl($C_1$-$C_5$)ammonium halide dissolved in an aprotic organic solvent.

31. The process of claim 28 characterized in that the base is potassium cyanide, the catalyst is a tetraalkyl-($C_1$-$C_5$)ammonium halide dissolved in an organic nitrile, and the aldehyde scavenger is an alkali metal metabisulfite.

**Patentansprüche**

1. Verfahren zur Umwandlung weniger pestizid wirksamer Isomere in einem Ausgangsgemisch eines kristallisierbaren Pyrethroids, wobei das Pyrethroid ein asymmetrisches Kohlenstoffatom, an welches ein epimerisierbares Proton gebunden ist, enthält, zu stärker pestizid wirksamen Isomeren,
gekennzeichnet durch:
(a) Herstellen einer Aufschlämmung des Ausgangsgemisches in einem flüssigen Medium, das im wesentlichen aus einem aliphatischen oder alicyclischen Kohlenwasserstoff mit 5 - 16 Kohlenstoffatomen besteht, in welchem die stärker pestizid wirksamen Isomere im wesentlichen unlöslich sind, wobei das Gewichtsverhältnis von Ausgangsgemisch zu Kohlenwasserstoff 1:1 bis 1:4 ist,
(b) Versetzen der Aufschlämmung mit einer Base und einem Katalysator, wobei der Katalysator im wesentlichen in dem flüssigen Medium löslich ist und ausgewählt wird aus einer quaternären Ammoniumverbindung, einer quaternären Phosphoniumverbindung und einem Kronenether, und

wobei das Gewichtsverhältnis von Ausgangsgemisch zu Base 10:1 oder höher und das Gewichtsverhältnis von Ausgangsgemisch zu Katalysator 10:1 bis 100:1 ist,
(c) Rühren des erhaltenen Gemisches unter Einhalten einer Temperatur im Bereich von 5 bis 35 °C, und
(d) Aufarbeiten der erhaltenen kristallisierten Isomere.

2. Verfahren nach Anspruch 1, gekennzeichnet dadurch, daß das Ausgangsgemisch die stärker wirksamen und weniger wirksamen Enantiomere von Cypermethrin umfaßt und das erhaltene kristalline Produkt überwiegend die stärker wirksamen Enantiomere umfaßt.

3. Verfahren nach Anspruch 2, gekennzeichnet dadurch, daß das Ausgangsgemisch die vier Enantiomerenpaare von Cypermethrin umfaßt und das erhaltene kristalline Produkt überwiegend die cis-2- und trans-2-Enantiomerenpaare umfaßt.

4. Verfahren nach Anspruch 2, gekennzeichnet dadurch, daß das Ausgangsgemisch die cis-1- und cis-2-Enantiomerenpaare von Cypermethrin umfaßt und das erhaltene kristalline Produkt überwiegend das cis-2-Enantiomerenpaar umfaßt.

5. Verfahren nach Anspruch 2, gekennzeichnet dadurch, daß das Ausgangsgemisch die trans-1- und trans-2-Enantiomerenpaare von Cypermethrin umfaßt und das erhaltene kristalline Produkt überwiegend das trans-2-Enantiomerenpaar umfaßt.

6. Verfahren nach Anspruch 1, gekennzeichnet dadurch, daß das Ausgangsgemisch die stärker wirksamen und weniger wirksamen Enantiomere von Cyfluthrin umfaßt und das erhaltene kristalline Produkt überwiegend die stärker wirksamen Enantiomere umfaßt.

7. Verfahren nach Anspruch 6, gekennzeichnet dadurch, daß das Ausgangsgemisch die vier Enantiomerenpaare von Cyfluthrin umfaßt und das erhaltene kristalline Produkt überwiegend die cis-2- und trans-2-Enantiomerenpaare umfaßt.

8. Verfahren nach Anspruch 6, gekennzeichnet dadurch, daß das Ausgangsgemisch die cis-1- und cis-2-Enantiomerenpaare von Cyfluthrin umfaßt und das erhaltene kristalline Produkt überwiegend das cis-2-Enantiomerenpaar umfaßt.

9. Verfahren nach Anspruch 6, gekennzeichnet dadurch, daß das Ausgangsgemisch die trans-1- und trans-2-Enantiomerenpaare von Cyfluthrin umfaßt und das erhaltene kristalline Produkt überwiegend das trans-2-Enantiomerenpaar umfaßt.

10. Verfahren nach Anspruch 1, gekennzeichnet dadurch, daß das Ausgangsgemisch die stärker wirksamen und weniger wirksamen Enantiomere von (Cyano)(3-phenoxyphenyl)methyl-3-(2,2-dibromethenyl)-2,2-dimethylcyclopropancarboxylat umfaßt und das erhaltene kristalline Produkt überwiegend die stärker wirksamen Enantiomere umfaßt.

11. Verfahren nach Anspruch 10, gekennzeichnet dadurch, daß das Ausgangsgemisch die 1R,cis-S- und 1R,cis-R-Isomere von (Cyano)(3-phenoxyphenyl)methyl-3-(2,2-dibromethenyl)-2,2-dimethylcyclopropancarboxylat umfaßt und das erhaltene Produkt überwiegend das 1R,cis-S-Isomere umfaßt.

12. Verfahren nach Anspruch 1, gekennzeichnet dadurch, daß die Base ein basisches Salz einer organischen oder anorganischen Säure ist.

13. Verfahren nach Anspruch 12, gekennzeichnet dadurch, daß die Base eine wässrige Lösung eines basischen Salzes einer organischen oder anorganischen Säure ist.

14. Verfahren nach Anspruch 12, gekennzeichnet dadurch, daß die Base ein Feststoff ist.

15. Verfahren nach Anspruch 1, gekennzeichnet dadurch, daß die Aufschlämmung in hohem Maße wasserfrei ist.

**16.** Verfahren nach Anspruch 1, gekennzeichnet dadurch, daß die Base ein festes Alkali- oder Erdalkalimetalloxid, -hydroxid, -carbonat, -bicarbonat, -cyanid, -cyanat, -acetat oder -borat, eine wässrige Lösung von einem oder mehreren davon oder ein Trialkylamin ist.

**17.** Verfahren nach Anspruch 1, gekennzeichnet dadurch, daß vor dem Kontakt mit der Aufschlämmung in Schritt (b) ein Addukt aus der Base und dem Katalysator erzeugt wird.

**18.** Verfahren nach Anspruch 1, gekennzeichnet dadurch, daß das Ausgangsgemisch die cis-1- und cis-2-Enantiomerenpaare von Cypermethrin umfaßt, das Lösungsmittel mindestens ein aliphatischer oder cycloaliphatischer Kohlenwasserstoff mit 5-16 Kohlenstoffatomen ist, die Base ein basisches Salz einer organischen oder anorganischen Säure ist und der Katalysator Tricaprylmethylammoniumchlorid ist.

**19.** Verfahren nach Anspruch 18, gekennzeichnet dadurch, daß das Lösungsmittel Heptan ist und das basische Salz ein Alkalimetallcarbonat oder -cyanid ist.

**20.** Verfahren nach Anspruch 1, gekennzeichnet dadurch, daß die Aufschlämmung durch Impfen einer Lösung des Ausgangsgemisches mit mindestens einem Kristall der stärker pestizid wirksamen Isomere erzeugt wird.

**21.** Verfahren nach Anspruch 1, gekennzeichnet dadurch, daß das Ausgangsgemisch die vier Enantiomerenpaare von Cypermethrin umfaßt, das Lösungsmittel mindestens ein aliphatischer oder cycloaliphatischer Kohlenwasserstoff mit 5-16 Kohlenstoffatomen ist, die Base ein basisches Salz einer organischen oder anorganischen Säure ist und der Katalysator Tricaprylmethylammoniumchlorid ist.

**22.** Verfahren nach Anspruch 21, gekennzeichnet dadurch, daß das Lösungsmittel Heptan ist und das basische Salz ein Alkalimetallcarbonat oder -cyanid ist.

**23.** Verfahren nach Anspruch 1, gekennzeichnet dadurch, daß das Ausgangsgemisch die trans-1- und trans-2-Enantiomerenpaare von Cypermethrin umfaßt, das Lösungsmittel mindestens ein aliphatischer oder cycloaliphatischer Kohlenwasserstoff mit 5-16 Kohlenstoffatomen ist, die Base ein basisches Salz einer organischen oder anorganischen Säure ist und der Katalysator Tricaprylmethylammoniumchlorid ist.

**24.** Verfahren nach Anspruch 23, gekennzeichnet dadurch, daß das Lösungsmittel Heptan ist und das basische Salz ein Alkalimetallcarbonat oder -cyanid ist.

**25.** Verfahren nach Anspruch 1, gekennzeichnet dadurch, daß das Ausgangsgemisch die vier Enantiomerenpaare von Cypermethrin oder Cyfluthrin umfaßt, wobei die Mengen der cis-2- und trans-2-Enantiomerenpaare jeweils von 15 bis 25 Gew.-% reichen und das kristalline Produkt mindestens jeweils 30 Gew.-% der cis-2- und trans-2-Enantiomerenpaare umfaßt.

**26.** Verfahren nach Anspruch 1, gekennzeichnet dadurch, daß das Ausgangsgemisch 20-80 Gew.-% des cis-1- und 80-20 Gew.-% des cis-2-Enantiomerenpaares von Cypermethrin oder Cyfluthrin umfaßt und das kristalline Produkt mindestens 30-90 Gew.-% des cis-2-Paares umfaßt.

**27.** Verfahren nach Anspruch 1, gekennzeichnet dadurch, daß das Ausgangsgemisch ein Gemisch aus 20-80 Gew.-% der trans-1- und 80-20 Gew.-% der trans-2-Enantiomeren von Cypermethrin oder Cyfluthrin umfaßt und das kristalline Produkt mindestens 30-90 Gew.-% des trans-2-Paares umfaßt.

**28.** Verfahren nach Anspruch 1, gekennzeichnet dadurch, daß in Schritt (b) die Aufschlämmung, die Base und Katalysator enthält, mit einem Aldehydfänger vereinigt wird.

**29.** Verfahren nach Anspruch 28, gekennzeichnet dadurch, daß der Aldehydfänger ein Alkalimetallmetabisulfit, -hydrogensulfit oder -hydrosulfit ist.

**30.** Verfahren nach Anspruch 28, gekennzeichnet dadurch, daß die Base ein Alkalimetallcyanid ist und der Katalysator ein in einem aprotischen organischen Lösungsmittel gelöstes Tetraalkyl($C_1$-$C_5$)-ammoniumhalogenid ist.

**31.** Verfahren nach Anspruch 28, gekennzeichnet dadurch, daß die Base Kaliumcyanid ist, der Katalysator ein in einem organischen Nitril gelöstes Tetraalkyl($C_1$-$C_5$)ammoniumhalogenid ist und der Aldehydfänger ein Alkalimetallmetabisulfit ist.

## Revendications

**1.** Procédé pour convertir des isomères moins actifs en tant que pesticides dans un mélange de départ d'un pyréthroide cristallisable ayant un carbone asymétrique auquel un proton épimérisable est attaché, en des isomères plus actifs en tant que pesticides,
caractérisé par :
(a) la formation d'une suspension du mélange de départ dans un milieu liquide constitué essentiellement d'un hydrocarbure aliphatique ou alicyclique de 5 - 16 atomes de carbone dans lequel les isomères plus actifs en tant que pesticides sont substantiellement insolubles, et le rapport en poids du mélange de départ à l'hydrocarbure est de 1 : 1 à 1 : 4,
(b) la mise en contact de la suspension avec une base et un catalyseur, ledit catalyseur étant substantiellement soluble dans le milieu liquide et choisi parmi un composé ammonium quaternaire, un composé phosphonium quaternaire et un éther couronne, et le rapport en poids du mélange de départ à la base étant de 10:1 ou supérieur et le rapport en poids du mélange de départ au catalyseur étant de 10:1 à 100:1,
(c) l'agitation du mélange obtenu en maintenant une température dans un éventail de 5 à 35 ° C, et
(d) la récupération des isomères résultants cristallisés.

**2.** Le procédé de la revendication 1 caractérisé en que le mélange de départ comprend les énantiomères plus actifs et moins actifs de la cyperméthrine et le produit résultant cristallin comprend principalement les énantiomères plus actifs.

**3.** Le procédé de la revendication 2 caractérisé en ce que le mélange de départ comprend les quatres paires d'énantiomères de la cyperméthrine et le produit cristallin résultant comprend principalement les paires d'énantiomères cis-2 et trans-2.

**4.** Le procédé de la revendication 2 caractérisé en ce que le mélange de départ comprend les paires d'énantiomères de la cyperméthrine cis-1 et cis-2 et le produit cristallin résultant comprend principalement la paire d'énantiomères cis-2.

**5.** Le procédé de la revendication 2 caractérisé en ce que le mélange de départ comprend les paires d'énantiomères trans-1 et trans-2 de la cvperméthrine et le produit cristallin résultant comprend principalement la paire d'énantiomères trans-2.

**6.** Le procédé de la revendication 1 caractérisé en ce que le mélange de départ comprend les énantiomères plus actifs et moins actifs de la cyfluthrine et le produit cristallin résultant comprend principalement les énantiomères plus actifs.

**7.** Le procédé de la revendication 6 caractérisé en ce que le mélange de départ comprend les quatre paires d'énantiomères de la cyfluthrine et le produit cristallin résultant comprend principalement les paires d'énantiomères cis-2 et trans-2.

**8.** Le procédé de la revendication 6 caractérisé en ce que le mélange de départ comprend les paires d'énantiomères cis-1 et cis-2 de la cyfluthrine et le produit cristallin résultant comprend principalement la paire d'énantiomères cis-2.

**9.** Le procédé de la revendication 6 caractérisé en ce que le mélange de départ comprend les paires d'énantiomères trans-1 et trans-2 de la cyfluthrine et le produit cristallin résultant comprend principalement la paire d'énantiomères trans-2.

**10.** Le procédé de la revendication 1 caractérisé en ce que le mélange de départ comprend les énantiomères plus actifs et moins actifs du 3-(2,2-dibromoéthényl)-2,2-diméthylcyclopropane-carboxylate de (cyano)(3-phénoxyphényl)méthyle et le produit cristallin résultant comprend principalement les énantiomères plus actifs.

**11.** Le procédé de la revendication 10 caractérisé en ce que le mélange de départ comprend les isomères 1R,cis S et 1R, cis R du 3-(2,2-dibromoéthényl)-2,2-diméthylcyclopropanecarboxylate de (cyano)(3-phénoxyphényl)méthyle et le produit cristallin résultant comprend principalement l'isomère 1R,cis S.

**12.** Le procédé de la revendication 1 caractérisé en ce que la base est un sel basique d'un acide organique ou inorganique.

**13.** Le procédé de la revendication 12 caractérisé en ce que la base est une solution aqueuse d'un sel basique d'un acide organique ou inorganique.

**14.** Le procédé de la revendication 12 caractérisé en ce que la base est un solide.

**15.** Le procédé de la revendication 1 caractérisé en ce que la suspension est considérablement anhydre.

**16.** Le procédé de la revendication 1 caractérisé en ce que la base est un oxyde, hydroxyde, carbonate, bicarbonate, cyanure, cyanate, acétate ou borate de métal alcalin ou alcalino-terreux solide, une solution aqueuse d'un ou de plusieurs d'entre eux, ou une trialkyl amine.

**17.** Le procédé de la revendication 1 caractérisé en ce qu'un mélange de la base et du catalyseur est formé avant la mise en contact avec la suspension dans l'étape (b).

**18.** Le procédé de la revendication 1 caractérisé en ce que le mélange de départ comprend les paires d'énantiomères cis-1 et cis-2 de la cyperméthrine, le solvant est au moins un hydrocarbure aliphatique ou cycloaliphatique de 5 à 16 atomes de carbone, la base est un sel basique d'un acide organique ou inorganique, et le catalyseur est le chlorure de tricaprylméthylammonium.

**19.** Le procédé de la revendication 18 caractérisé en ce que le solvant est l'heptane et le sel basique est un carbonate ou cyanure d'un métal alcalin.

**20.** Le procédé de la revendication 1 caractérisé en ce que la suspension est formé en ensemençant une solution du mélange de départ avec au moins un crystal des isomères les plus actifs en tant que pesticides.

**21.** Le procédé de la revendication 1 caractérisé en ce que le mélange de départ comprend les quatres paires d'énantiomères de la cyperméthrine, le solvant est au moins un hydrocarbure aliphatique ou cycloaliphatique de 5 à 16 atomes de carbone, la base est un sel basique d'un acide organique ou inorganique, et le catalyseur est le chlorure de tricaprylméthylammonium.

**22.** Le procédé de la revendication 21 caractérisé en ce que le solvant est l'heptane et le sel basique est un carbonate ou cyanure d'un métal alcalin.

**23.** Le procédé de la revendication 1 caractérisé en ce que le mélange de départ comprend les paires d'énantiomères trans-1 et trans-2 de la cyperméthrine, le solvant est au moins un hydrocarbure aliphatique ou cycloaliphatique de 5 à 16 atomes de carbone, la base est un sel basique d'un acide organique ou inorganique, et le catalyseur est le chlorure de tricaprylméthylammonium.

**24.** Le procédé de la revendication 23 caractérisé en ce que le solvant est l'heptane et le sel basique est un carbonate ou cyanure d'un métal alcalin.

**25.** Le procédé de la revendication 1 caractérisé en ce que le mélange de départ comprend les quatres paires d'énantiomères de la cyperméthrine ou de la cyfluthrine dans lequel les proportions de chacune des paires d'énantiomères cis-2 et trans-2 varient de 15 à 25 % en poids et le produit cristallin comprend au moins 30% en poids de chacune des paires d'énantiomères cis-2 et trans-2.

**26.** Le procédé de la revendication 1 caractérisé en ce que le mélange de départ comprend 20-80 % en poids de la paire d'énantiomères cis-1 et 80-20% en poids de la paire d'énantiomères cis-2 de la cvpermethrine ou de la cyfluthrine et le produit cristallin comprend au moins 30-90 % en poids de la paire cis-2.

**27.** Le procédé de la revendication 1 caractérisé en ce que le mélange de départ comprend un mélange de 20-80% en poids d'énantiomères trans-1 et 80-20% en poids d'énantiomères trans-2 de la cyperméthrine ou de la cyfluthrine et le produit cristallin comprend au moins 30-90% en poids de la paire trans-2.

**28.** Le procédé de la revendication 1 caractérisé en ce que, dans l'étape (b), la suspension contenant la base et le catalyseur est mise en contact avec un agent piégeur d'aldéhyde.

**29.** Le procédé de la revendication 28 caractérisé en ce que l'agent piégeur d'aldéhyde est un métabisulfite, un hydrogénosulfite ou un hydrosulfite d'un métal alcalin.

**30.** Le procédé de la revendication 28 caractérisé en ce que la base est un cyanure d'un métal alcalin et le catalyseur est un halogénure de tétraalkylammonium (C1-C5) dissous dans un solvant organique aprotique.

**31.** Le procédé de la revendication 28 caractérisé en ce que la base est un cyanure de potassium, le catalyseur est un halogénure de tétraalkylammonium (C1-C5) dissous dans un nitrile organique, et l'agent piégeur d'aldéhyde est un métabisulfite d'un métal alcalin.